(19) Europäisches Patentamt
European Patent Office
Office européen des brevets

(11) Publication number : **0 416 804 B1**

# (12) EUROPEAN PATENT SPECIFICATION

(45) Date of publication of patent specification : **26.05.93 Bulletin 93/21**

(51) Int. Cl.$^5$ : **A61K 31/18,** A61K 9/08, A61K 47/14, A61K 47/22

(21) Application number : **90309439.9**

(22) Date of filing : **29.08.90**

## (54) External preparation containing amusulosin.

(30) Priority : **04.09.89 JP 227575/89**

(43) Date of publication of application : **13.03.91 Bulletin 91/11**

(45) Publication of the grant of the patent : **26.05.93 Bulletin 93/21**

(84) Designated Contracting States : **AT BE CH DE DK ES FR GB GR IT LI LU NL SE**

(56) References cited :
**EP-A- 0 027 286**
**EP-A- 0 034 432**
**EP-A- 0 182 635**
**EP-A- 0 194 838**

(73) Proprietor : **YAMANOUCHI PHARMACEUTICAL CO. LTD.**
**No. 3-11 Nihonbashi-Honcho, 2-chome**
**Chuo-ku**
**Tokyo (JP)**

(72) Inventor : **Konno, Yutaka**
**974-6 Sangamyo**
**Yaizu-shi, Shizuoka 425 (JP)**
Inventor : **Katsuma, Masataka**
**Rm.201 No.1 Bldg. Surugadaidanchi, 1-3**
**Surugadai**
**Fujieda-shi, Shizuoka 426 (JP)**

(74) Representative : **Geering, Keith Edwin et al**
**REDDIE & GROSE 16 Theobalds Road**
**London WC1X 8PL (GB)**

EP 0 416 804 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a therapeutic preparation containing 5-[2-[[2-(o-ethoxyphenoxy)-ethyl]amino]propyl]-2-methoxybenzenesulfonamide or salt thereof as an active ingredient, for external administration useful for the improvement of dysuria and lower urinary tract disease.

5-[2-[[2-(o-Ethoxyphenoxy)-ethyl]amino]propyl]-2-methoxybenzenesulfonamide is a phenethylamine derivative of the following formula (I).

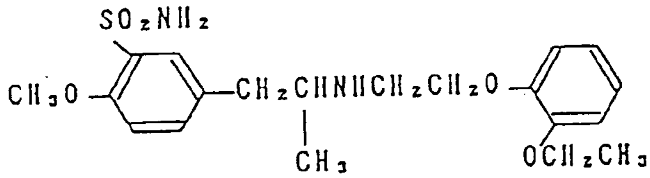 (I)

This compound, alias amusulosin, has potent and selective $\alpha_1$-receptor blocking activity (cf. EP-A-O 034 432). The $\alpha_1$-receptor blocking activity of amusulosin is higher than that of prazosin hydrochloride and, as a pharmacological action, reduces the contraction of the rabbit prostate, urethra and vesical triangle (trigone of the bladder) and of the human urethra and vesical triangle. Therefore, amusulosin can be clinically useful as an ameliorating agent for certain diseases such as dysuria and lower urinary tract disease. However, as these diseases occur more frequently in subjects of advanced age, the development of a sustained release preparation such as a long-acting oral preparation or a transdermal therapeutic system (hereinafter, referred to briefly as TTS) has been keenly demanded.

Elderly patients, particularly those bed-ridden, cannot be easily medicated by the oral route and, therefore, the advent of a TTS preparation, which may be administered more expediently than an oral preparation, is in demand. The demand for TTS is fortified by the fact that a TTS preparation is not subject to the first-pass effect, which is inevitable with an oral drug, that it insures sustained efficacy and that it can be withdrawn at any time.

However, since the permeability of the skin to most drugs is low, only limited kinds of drugs have heretofore been available in preparations of this type.

Furthermore, since the epidermal tissue acts as a barrier to prevent ingress of foreign matter, the absorption of a drug through the skin is not only greatly dependent on the intrinsic properties of the drug but also subject to the interaction between the drug and the remainder of the pharmaceutical composition and the recipient skin. Therefore, a TTS assuring increased transdermal absorption of one drug can hardly be predicted from the TTS technology established for another drug.

The same was true with amusulosin or a salt thereof, for which an external preparation useful for TTS was keenly demanded, and it was actually very difficult to design and manufacture an external preparation which would prove clinically useful.

As transdermal absorption promoting agent, a number of compounds have heretofore been proposed. These include 1-alkylazacycloheptan-2-ones, cis-olefins, aliphatic dicarboxylic acid diesters (e.g. diethyl sebacate etc.), nicotinic acid esters and so on (cf. U.S. Patents 3,989,816 and 4,390,520 and EP-A-271983).

It was known that amusulosin hydrochloride is only sparingly soluble in water and pure alcohol. We were therefore surprised that this compound showed no less than 10-fold greater solubility in aqueous alcohol.

As a result of extensive investigation on the transdermal absorption promoting activity of a large number of compounds to develop a TTS preparation of amusulosin hydrochloride, we have found that aliphatic dicarboxylic acid diesters, nicotinic acid esters and isonicotinic acid esters, used either independently or in combination or together with a terpene, are able to remarkably promote the transdermal absorption of amusulosin hydrochloride. It was surprising that the transdermal absorption promoting effect of a combination of amusulosin hydrochloride with such transdermal absorption promoting agent is by far greater in degree than that of the conventional combination of a drug with a transdermal absorption promoting agent.

It has been thus found that the use of aqueous alcohol increases the solubility of the active compounds of the present invention (amusulosin or a salt thereof) to a remarkable extent and, thus, facilitates the manufacture of pharmaceutical products. It was also found that the addition of a certain kind of ester results in a marked improvement in the transdermal penetration of the active compound.

The present invention is, therefore, directed to an external preparation which contains aqueous alcohol and certain esters (hereinafter, referred to as a transdermal absorption promoting agent) as additives.

Amusulosin having the above formula (I) has excellent $\alpha$-adrenaline receptor ($\alpha_1$ receptor) blocking activity and can be utilized clinically as an ameliorating agent for dysuria and lower urinary tract diseases as mentioned

above. It can also be of value as a therapeutic agent for hypertension, congestive cardioplegia, angina pectoris, prostatic hypertrophy, chromophilic cytoma and peripheral vascular disorder.

The aforementioned salt of amusulosin include pharmaceutically acceptable salts, such as addition salts with inorganic acids (e.g. hydrochloric acid, sulfuric acid, hydrobromic acid) and with organic acids (e.g. acetic acid, oxalic acid, maleic acid, fumaric acid, citric acid, lactic acid). Particularly useful for clinical application is the hydrochloride, namely amusulosin hydrochloride (hereinafter referred as to AB-250, m.p. 254-256°C).

Amusulosin shown by formula (I) has one asymmetric carbon atom and there can exist isomers due to the presence of such a carbon atom. Such isomers all fall within the scope of the present invention either in each individual isolated form or in a mixture form. The (R)-form isomer is most preferable.

In the present invention, one or more amusulosin compounds is or are used in a total proportion of 0.5 - 10, preferably 1 - 5, wt.% based on the total pharmaceutical composition. Herein percentages are by weight unless otherwise indicated.

The alcohol to be used in the present invention is preferably a lower alcohol such as methanol, ethanol, propanol, isopropanol and butanol, more preferably ethanol or isopropanol, most preferably ethanol. The alcohol content is preferably of the order of 40 to 60 percent, for the solubility of the compound is sharply increased in this range.

The preferred proportion of the aqueous alcohol is dependent on the amount of the above compound and the dosage form of the pharmaceutical composition but is from 2.5 to 98.5 percent by weight and more desirably from 5 to 97 percent by weight.

The ester to be used as the transdermal absorption promoting agent in the present invention is selected from among nicotinic acid esters, isonicotinic acid esters and aliphatic dicarboxylic acid diesters, mixtures of such esters, with or without addition of terpenes, more preferably aliphatic dicarboxylic acid diesters.

Examples of the aliphatic dicarboxylic acid diesters include the diesters of such aliphatic dicarboxylic acids as succinic acid, adipic acid, suberic acid, sebacic acid and maleic acid with lower alcohols of 1 to 6 carbon atoms such as methanol, ethanol, propanol, isopropanol, butanol, tert-butanol, pentanol and hexanol.

The alcohol residues of nicotinic acid esters or isonicotinic acid esters include the above-mentioned alcohols and benzyl alcohol.

Since some of these absorption promoting agents, such as aliphatic dicarboxylic acid diesters, are considered to be concentration-dependent, the proportion of the absorption promoting agent must be determined in consideration of its kind. Usually, however, the transdermal absorption agent can be used in a proportion of 1 to 30 percent,and preferably 2 to 20 percent, based on the total composition.

Examples of terpenes which may be added as an optional component to the present external preparation include peppermint oil, orange oil, turpentine oil, ℓ-menthol and d-limonene.

The dosage form for the external preparation according to the present invention includes all preparations designed for application to the skin and capable of producing pharmacological effects locally or systemically, thus including such varied dosage forms as solutions in aqueous alcohols, spray dosage forms, gel ointments (creams) based on carboxy-vinyl polymers, general ointments, lotions, emulsions and cataplasms.

The preparation for external administration according to the present invention can be processed into a final product by selective use of a carrier of base optimal for the intended dosage form. The carrier or base may be selected from among those commonly employed which do not interfere with the solubility and transdermal penetration of the drug compound. The external preparation of the invention is best provided in the weakly alkaline to neutral pH range of from 5.5 to 8.5 and preferably 6 to 8.

The external preparation of the invention can be manufactured by dissolving the active compound in the aqueous alcohol and by adding the transdermal absorption promoting agent to the resultant solution with stirring to give homogeneous solution.

To ensure the homogeneity of the pharmaceutical composition, there may be incorporated, in suitable proportions, various emulsifiers such as polyoxyethylene-hydrogenated castor oil derivative (e.g. Nikkol HCO-60, trade name), polyoxyethylene-polyoxypropylene glycol ether (e.g. Pluronic F68, trade name), polyoxyethylene sorbitan monolaurate (Tween 20, trade name), and polyoxyethylene monolaurate (Nikkol MYL-10, trade name).

In addition, ethylene glycol, propylene glycol, or triethylene glycol may be further incorporated for the purpose of preventing precipitation of crystals and accelerating the transdermal penetration of the active compound.

It is also possible to use white petrolatum, silicone oil (e.g. Silicone Fluid 360, trade name), gelatin, carboxyvinyl polymer, polyvinyl alcohol, or (meth)acrylic acid-(meth)acrylate copolymer, ointment bases such as liquid paraffin., humectants such as glycerin, and/or preservatives such as paraben as formulating agents.

The dosage of the active component of the present preparation differs according to the kind of preparation, the age, weight, and symptom of a patient, but is usually 0.01 to 6 mg/day, preferably 0.1 to 0.6 mg/day.

The present invention will now be illustrated in greater detail by way of Test Example and Examples.

TEST EXAMPLE

1. Data on the solubility increasing effect of aqueous alcohol:

The solubility of AB-250 in water of pH 5-7 was 0.4% and that in ethanol was 0.03%.

In contrast, the solubility of the same compound in aqueous ethanol containing 60% of ethanol (Michaelis buffer containing 60% of ethanol, pH 7) was 4.8% and that in aqueous ethanol containing 50% of ethanol (Michaelis buffer containing 50% of ethanol, pH 8) was 5.1%. It is, thus, evident that the use of aqueous ethanol results in a remarkable increase in the solubility of the active compound.

More or less the same effect was obtained when isopropyl alcohol was used in lieu of ethanol.

2. Data on the improving effect on percutaneous absorption:

An in vitro isolated guinea pig skin penetration test was performed using AB-250.

(1) Preparation of samples:

Thus, AB-250 was dissolved, with warming (about 50°C), in the mixtures of ethanol with Michaelis buffer which are indicated in Table 1. Then, the transdermal absorption promoting agent or the like as indicated in Table 1 was added and mixed well as the third ingredient to prepare samples.

(2) Determination of transdermal penetration rate (flux):

From the abdominal region of the male Hartley guinea pig (6-7 weeks old), with hair clipped the previous day, the skin was isolated and mounted on a Franz diffusion cell.

Each AB-250 sample was added to the donor side and isotonized 10 mM potassium dihydrogen phosphate solution was added to the receptor side. The experiment was performed at a cell temperature of 37°C.

The receptor solution was sampled at 2-hour intervals up to 8 hours and the concentration of AB-250 in each sample solution was measured by high performance liquid chromatography (HPLC). The cumulative transdermal penetration amounts ($\mu g/cm^2$) of AB-250 at hours 2, 4, 6 and 8 were respectively determined and plotted on the ordinate while the time was plotted on the abscissa. From the resulting correlogram, the gradient was calculated and expressed as flux (penetration rate per unit area, $\mu g/cm^2/h$).

This experiment was carried out in an open system and a closed system.

(3) Results:

The fluxes and flux ratios found in the above experiment are shown in Table 1.

Table 1

| Formulation | | Proportions of Ingredients (w/w%) | Flux ($\mu g/cm^2/h$) | Flux ratio |
|---|---|---|---|---|
| (i) Open system | | | | |
| | *AB-250/EtOH/pH 8 Buffer | (5/47.5/47.5) | 0.4 | 1 |
| | *AB-250/EtOH/pH 7 Buffer | (5/47.5/47.5) | 5.2 | 13 |
| | AB-250/EtOH/pH 8 Buffer/DID | (5/45/45/5) | 35.5 | 89 |
| | AB-250/EtOH/pH 7 Buffer/DID | (5/45/45/5) | 89.1 | 223 |
| | AB-250/EtOH/pH 7 Buffer/DES | (5/45/45/5) | 146.5 | 366 |
| | AB-250/EtOH/pH 7 Buffer/DES | (1/47/47/5) | 108.2 | 271 |
| | AB-250/EtOH/pH 7 Buffer/DES | (1/49/49/1) | 8.3 | 21 |
| | AB-250/EtOH/pH 7 Buffer/DES | (1/48.25/48.25/2.5) | 27.3 | 68 |
| | AB-250/EtOH/pH 7 Buffer/DES/INB | (5/40/40/5/10) | 814.9 | 2037 |
| | AB-250/EtOH/pH 7 buffer/DES/PG | (2/44/44/5/5) | 143.7 | 359 |
| (ii) Closed system | | | | |
| | AB-250/EtOH/pH 7 Buffer/DES | (5/45/45/5) | 426.7 | |

note: "*" is control.

In Table 1, DID stands for diisopropyl adipate, DES for diethyl sebacate, INB for n-butyl isonicotinate, and PG for propylene glycol.

The above experiment indicated that the combined use of aqueous ethanol (ethanol-containing buffer) and the transdermal absorption promoting agent results in a remarkable increase in the flux value.

With regard to the effect of pH, higher transdermal penetration was achieved at pH 7 than at pH 8. With regard to the transdermal absorption promoting agent, INB and DES were more effective than DID. In the comparison between the open and the closed system, the closed system was conducive to relative higher transdermal penetration.

EXAMPLE 1 (Spray Dosage Form)

To a mixed solution of 2.0 g of AB-250, 46.5 g of ethanol and 46.5 g of Michaelis buffer (pH 7) was added 5.0 g of DES with stirring to give a homogeneous AB-250 solution. A pressure container (15 ml capacity, about 0.05 ml delivery per actuation) was filled with 10 ml of the above obtained solution to provide a spray dosage form.

EXAMPLE 2 (Spray Dosage Form)

To a mixed solution of 2.0 g of AB-250, 44.0 g of ethanol, 44.0 g of Michaelis buffer (pH 7) and 5.0 g of PG was added 5.0 g of DES with thorough stirring to give a homogeneous AB0-250 solution. Thereafter, the same procedure as described in Example 1 was followed to provide a spray dosage form.

EXAMPLE 3 (Gel Ointment)

Using 30 g of purified water, 1.0 g of Carbopol 940 was swollen with stirring. Then, 0.5 g of diisopropanolamine was added thereto for gelation. A mixed solution of 3.0 g of AB-250, 46 g of ethanol and 46 g of Michaelis buffer (pH 7) was prepared and 5 g of DES was added thereto and mixed well to give a homogeneous solution. Then, 68.5 g of this solution was gradually added to the Carbopol gel prepared above with constant stirring and the mixture was well kneaded to give a homogeneous gel ointment.

EXAMPLE 4

To a mixed solution of 1.0 go AB-250, 48.6 g of ethanol and 32.4 g of Michaelis buffer (pH 7) was added 5.0 g of DES and the mixture was stirred well to give a homogeneous AB-250 solution. To this solution were added 10.0 g of triethylene glycol and 3.0 g of oil of peppermint and the mixture was stirred well to provide a homogeneous solution of AB-250.

## Claims

### Claims for the following Contracting States : AT, BE, CH, DE, DK, FR, GB, IT, LI, LU, NL, SE

1. An preparation for external administration which comprises:
   1) at least one compound selected from 5-[2-[[2-(o-ethoxyphenoxy)-ethyl]amino]propyl]-2-methoxybenzenesulfonamide and salts thereof as an active ingredient,
   2) aqueous alcohol, and
   3) at least one transdermal absorption promoting agent selected from aliphatic dicarboxylic acid diesters, nicotinic acid esters and isonicotinic acid esters, with or without addition of a terpene.

2. A preparation according to claim 1 wherein said aqueous alcohol is an aqueous ethanol.

3. A preparation according to claim 1 or 2 wherein said aqueous alcohol contains 40 to 60% by weight of alcohol.

4. A preparation according to any preceding claim wherein said transdermal absorption promoting agent comprises aliphatic dicarboxylic acid diester.

5. A preparation according to claim 4 wherein said aliphatic dicarboxylic acid diester comprises diethyl sebacate.

6. A preparation according to claim 5 wherein said transdermal absorption promoting agent comprises a mixture of diethyl sebacate and n-butyl isonicotinate.

**Claims for the following Contracting States : ES, GR**

1. A method of preparing preparation for external administration which comprises dissolving at least one compound selected from 5-[2-[[2-(o-ethoxyphenoxy)-ethyl]amino]propyl]-2-methoxybenzen esulfonamide and salts thereof as an active ingredient in aqueous alcohol, and adding at least one transdermal absorption promoting agent selected from aliphatic dicarboxylic acid diesters, nicotinic acid esters and isonicotinic acid esters, with or without addition of a terpene, with stirring to give an homogeneous solution.

2. A method according to claim 1 wherein said aqueous alcohol is an aqueous ethanol.

3. A method according to claim 1 or 2 wherein said aqueous alcohol contains 40 to 60% by weight of alcohol.

4. A method according to any preceding claim wherein said transdermal absorption promoting agent comprises aliphatic dicarboxylic acid diester.

5. A method according to claim 4 wherein said aliphatic dicarboxylic acid diester comprises diethyl sebacate.

6. A method according to claim 5 wherein said transdermal absorption promoting agent comprises a mixture of diethyl sebacate and n-butyl isonicotinate.

**Patentansprüche**

**Patentansprüche für folgende Verstragsstaaten : AT, BE, CH, DE, DK, FR, GB, IT, LI, LU, NL, SE**

1. Eine Zubereitung zur äußerlichen Verabreichung, welche umfaßt:
   1) wenigstens eine Verbindung, ausgewählt aus 5-[2-[[2-(o-Ethoxyphenoxy)-ethyl]amino]propyl]-2-methoxybenzensulfonamid und deren Salzen als ein aktiver Bestandteil,
   2) wäßrigen Alkohol und
   3) wenigstens ein die transdermale Absorption förderndes Mittel, ausgewählt aus aliphatischen Dicarbonsäurediestern, Nicotinsäureestern und Isonicotinsäureestern, mit oder ohne Zusatz von Terpen.

2. Zubereitung gemäß Anspruch 1, worin der wäßrige Alkohol wäßriges Ethanol ist.

3. Zubereitung gemäß Anspruch 1 oder 2, worin der wäßrige Alkohol 40 bis 60 Gew.-% Alkohol enthält.

4. Zubereitung gemäß irgendeinem der vorhergehenden Ansprüche, worin das die transdermale Absorption fördernde Mittel aliphatische Dicarbonsäurediester umfaßt.

5. Zubereitung gemäß Anspruch 4, worin der aliphatische Dicarbonsäurediester Diethylsebacinat umfaßt.

6. Zubereitung gemäß Anspruch 5, worin das die transdermale Absorption fördernde Mittel eine Mischung von Diethylsebacinat und n-Butylisonicotinat umfaßt.

**Patentansprüche für folgende Verstragsstaaten : ES, GR**

1. Verfahren zur Herstellung einer Zubereitung zur äußerlichen Verabreichung, welches umfaßt die Auflösung einer Verbindung, ausgewählt aus 5-[2-[[2-(o-Ethoxyphenoxy)-ethyl]amino]propyl]-2-methoxybenzensulfonamid und deren Salzen als ein aktiver Bestandteil, in wäßrigem Alkohol und Zugabe von wenigstens einem die transdermale Absorption fördernden Mittel, ausgewählt aus aliphatischen Dicarbonsäurediestern, Nicotinsäureestern und Isonicotinsäureestern, mit oder ohne Zusatz von Terpen,

unter Rühren, wobei eine homogene Lösung erhalten wird.

2. Verfahren nach Anspruch 1, worin der wäßrige Alkohol wäßriges Ethanol ist.

3. Verfahren nach Anspruch 1 oder 2, worin der wäßrige Alkohol 40 bis 60 Gew.-% Alkohol enthält.

4. Verfahren nach irgendeinem der vorhergehenden Ansprüche, worin das die transdermale Absorption fördernde Mittel aliphatische Dicarbonsäurediester umfaßt.

5. Verfahren nach Anspruch 4, worin der aliphatische Dicarbonsäurediester Diethylsebacinat umfaßt.

6. Veerfahren nach Anspruch 5, worin das die transdermale Absorption fördernde Mittel eine Mischung von Diethylsebacinat und n-Butylisonicotinat umfaßt.

## Revendications

### Revendications pour les Etats contractants suivants : AT, BE, CH, DE, DK, FR, GB, IT, LI, LU, NL, SE

1. Une préparation pour une administration externe qui comprend:
   1) au moins un composé choisi à partir du 5-[2-[[2-(o-éthoxyphénoxy)-éthyl]amino]propyl]-2-méthoxybenzènesulfonamide et de ses sels comme principe actif,
   2) un alcool aqueux, et
   3) au moins un agent favorisant l'absorption transdermique choisi à partir des diesters d'acides dicarboxyliques aliphatiques, des esters d'acide nicotinique et des esters d'acide isonicotinique, avec ou sans addition d'un terpène.

2. Une préparation selon la revendication 1, dans laquelle ledit alcool aqueux est un éthanol aqueux.

3. Une préparation selon la revendication 1 ou 2, dans laquelle ledit alcool aqueux contient 40 à 60 % en poids d'alcool.

4. Une préparation selon l'une quelconque des revendications précédentes, dans laquelle ledit agent favorisant l'absorption transdermique comprend un diester d'acide dicarboxylique aliphatique.

5. Une préparation selon la revendication 4, dans laquelle ledit diester d'acide dicarboxylique aliphatique comprend du sébacate de diéthyle.

6. Une préparation selon la revendication 5, dans laquelle ledit agent favorisant l'absorption transdermique comprend un mélange de sébacate de diéthyle et d'isonicotinate de n-buthyle.

### Revendications pour les Etats contractants suivants : ES, GR

1. Un procédé pour préparer une préparation externe qui comprend la dissolution d'au moins un composé choisi parmi le 5-[2-[[2-(o-éthoxyphénoxy)-éthyl]amino]propyl]méthoxybenzènesulfonamide et ses sels comme principe actif dans un alcool aqueux, et l'addition d'au moins un agent favorisant l'absorption transdermique choisi parmi les diesters d'acides dicarboxyliques aliphatiques, les esters d'acide nicotinique et les esters d'acide isonicotinique, avec ou sans addition d'un terpène, en agitant pour former une solution homogène.

2. Un procédé selon la revendication 1, dans lequel ledit alcool aqueux est un éthanol aqueux.

3. Un procédé selon la revendication 1 ou 2, dans lequel ledit alcool aqueux contient 40 à 60 % en poids d'alcool.

4. Un procédé selon l'une quelconque des revendications précédentes, dans lequel ledit agent favorisant l'absorption transdermique comprend un diester d'acide dicarboxylique aliphatique.

5. Un procédé selon la revendication 4, dans lequel ledit diester d'acide dicarboxylique aliphatique comprend

du sébacate de diéthyle.

6. Un procédé selon la revendication 5, dans lequel ledit agent favorisant l'absorption transdermique comprend un mélange de sébacate de diéthyle et d'isonicotinate de n-buthyle.